(19)

**Europäisches Patentamt**
**European Patent Office**
**Office européen des brevets**

(11) **EP 4 052 649 A1**

(12) **EUROPEAN PATENT APPLICATION**

(43) Date of publication:
**07.09.2022 Bulletin 2022/36**

(21) Application number: **22150339.4**

(22) Date of filing: **10.08.2018**

(51) International Patent Classification (IPC):
**A61B 5/27** *(2021.01)*    **A61B 5/00** *(2006.01)*
**A01K 29/00** *(2006.01)*    A61D 9/00 *(2006.01)*

(52) Cooperative Patent Classification (CPC):
**A61B 5/6804; A01K 29/005; A61B 5/27;**
A61B 2503/40; A61B 2562/0209; A61D 9/00

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.08.2017   JP 2017157048
21.11.2017   JP 2017223991
21.11.2017   JP 2017223990**

(62) Document number(s) of the earlier application(s) in
accordance with Art. 76 EPC:
**18846930.8 / 3 669 769**

(71) Applicant: **TOYOBO CO., LTD.
Osaka-shi
Osaka 5300001 (JP)**

(72) Inventors:
• **KOMATSU, Yoko
Shiga, 5200292 (JP)**
• **KWON, Euichul
Shiga, 5200292 (JP)**
• **SEGUCHI, Maki
Shiga, 5200292 (JP)**

(74) Representative: **Müller-Boré & Partner
Patentanwälte PartG mbB
Friedenheimer Brücke 21
80639 München (DE)**

Remarks:
This application was filed on 05.01.2022 as a
divisional application to the application mentioned
under INID code 62.

(54) **GARMENT FOR MEASURING BIOLOGICAL INFORMATION AND BIOLOGICAL INFORMATION
MEASUREMENT METHOD**

(57)    The present invention relates to a garment (800) for measuring biological information for an animal, such as a dog, wherein the garment comprises a body contact electrode configured to be in contact with a back side, especially a spinous process, a rib, or a scapula of the animal when the garment is worn by the animal. Such a positioning of the electrode allows for a stable biological measurement because, even if the animal is lying on its belly or on its side, the electrode is not sandwiched between the animal and the floor, so that a displacement of the electrode is less likely to occur.

[Fig. 6]

EP 4 052 649 A1

**Description**

TECHNICAL FIELD

The present invention relates to an electrode member for measuring biological information, a biological information measurement device, an attachment method for electrode member for measuring biological information, and a biological information measurement method. A living body in the present invention indicates a living body belonging to the animal world, preferably including all vertebrates, and more preferably vertebrates living mainly on land.

More particularly, the present invention relates to a detachable electrode member in which an electrode for measuring biological information can be attached at an optimum position so that the electrode is brought into close contact with a body. More specifically, the present invention relates to an electrode member, for measuring biological information, which can be used for existing harnesses, belts, garments, etc. (hereinafter collectively referred to as clothes), enables to determine an optimum position for achieving close contact of an electrode for measuring biological information with a body, enables the electrode to be attached to the optimum position, and can provide high measurement accuracy.

BACKGROUND ART

In recent years, the number of owners who place importance on the health of pets has increased, and the need for easily acquiring biological information of pets has increased. When a sensor (electrode) for monitoring biological information is closely attached to the body of an animal covered with hair, such as dogs, cats, rabbits, goats, miniature pigs, or horses, the owner needs to split the hair as much as possible or to trim the hair. Patent Document 1 discloses an invention of a jacket which can satisfactorily perform measurement on an electrocardiogram for a long time without giving a feeling of discomfort to dogs. Specifically, in the jacket, an electrode is attached to a detection site using an adhesive pad, and further, the electrode is appropriately pressed by externally applying a force using a fastening tool, in order to eliminate a need to trim an area where a sensor (electrode) for monitoring biological information is to be attached, or in order that it is only necessary to trim the minimum area approved by an owner or the like even if trimming is required.

Patent Document 2 discloses an invention of a wearable instrument that can measure biological information for a long time by pressing an electrode against the axilla of an animal. In the biological information measurement instrument, the length of a pull-up band shaped like a strap can be adjusted so that a measurement section is in close contact with the axilla of the animal. When the instrument is put on a dog, the measurement section is likely to be easily in close contact with the body, because the density of hair is usually low on the axilla.

However, in both inventions, the electrode is placed in the lower part of the chest, such as the axilla, so that, when the dog is lying on its belly, the electrode or device is sandwiched between the dog and the floor and is strongly pressed against the body. This arises the problems that the electrode is likely to be displaced, the dog may express a feeling of discomfort, and the skin in the area where the electrode and the device are in strong contact may be damaged.

Further, when it is assumed that pet animals are target living bodies to be measured, the living bodies widely range in size from small-sized animals such as small birds, guinea pigs, or Chihuahuas to large dogs. Furthermore, when it is assumed that animals in zoos or wild animals are monitored and examined, it is necessary to consider application of such electrode and device to birds, reptiles such as lizards, snakes, turtles, or iguanas, amphibians such as salamanders, or frogs, and super-sized animals such as wild cattle, elephants, giraffes, or whales. Moreover, the optimal electrode positions for measurement of biological information differ according to kinds and individual differences of animals. There is no universal wearable biological information measuring device, and it should be noted that designing and manufacturing wearable clothes for measuring biological information for each animal are extremely difficult in terms of size and electrode position from economical and rational viewpoints.

PRIOR ART DOCUMENTS

PATENT DOCUMENTS

Patent Document 1: JP-A-2006-141467
Patent Document 2: WO 2017/026416

SUMMARY OF THE INVENTION

PROBLEMS TO BE SOLVED BY THE INVENTION

The present invention has been made in view of the problems of prior arts described above. That is, an object of the present invention is to implement an electrode member, for measuring biological information, which can be attached to existing harnesses, belts, garments, etc.
(hereinafter collectively referred to as clothes) in a detachable manner, enables to determine an optimum position for achieving close contact of an electrode for measuring biological information with a body, enables the electrode to be attached to the optimum position, and can provide high measurement accuracy. When the electrode member is used, the electrode for measuring biological information can be attached at the optimum position for achieving close contact of the electrode with the body regardless of the size of the body, whereby biological information can be measured with high measurement accuracy.

SOLUTIONS TO THE PROBLEMS

As a result of intensive studies, the present inventors have found that the above problems can be solved by the following means, and have reached the present invention.
That is, the present invention includes the following configurations.

[1] An electrode member for measuring biological information, the electrode member comprising, at least:

a flexible substrate;
a single electrode section formed from a flexible conductive material on a first surface of the flexible substrate;
a single-pole connector section on the first surface; and
a wiring electrically connecting the electrode section and the connector section on the first surface,
the electrode member being provided with a member for attaching the electrode member to clothes on a second surface of the flexible substrate.

[2] The electrode member for measuring biological information according to [1],
wherein the flexible conductive material is a stretchable conductor formed from at least a conductive filler and a flexible resin.
[3] The electrode member for measuring biological information according to [1],
wherein the flexible conductive material is a conductive fabric.
[4] The electrode member for measuring biological information according to any one of [1] to [3],
wherein the electrode section is made of a material same as a material of the wiring.
[5] The electrode member for measuring biological information according to any one of [1] to [4],
wherein the member for attaching the electrode member to clothes is a hook member of a hook-and-loop fastener.
[6] A biological information measurement device comprising:

an electronic device having at least a potential measurement function and a function for storing and/or transmitting a measured result to an outside; and
a plurality of the electrode members for measuring biological information according to any one of [1] to [5] connected to the electronic device,
the biological information measurement device measuring a biopotential of a living body.

[7] An attachment method for an electrode member for measuring biological information for attaching the electrode member for measuring biological information according to any one of [1] to [5] to an edge of clothes worn by a living body that is a subject, the attachment method comprising:

folding the electrode member in a direction in which the second surface comes in contact with the clothes; and
wrapping the edge with the electrode section being positioned on a side in contact with the living body and the connector section being positioned on an outside of the clothes.

[8] A biological information measurement method comprising:
measuring biological information of an animal using an electrode member for measuring biological information attached to clothes by at least the method according to [7].
[9] A garment for measuring biological information for animals, the garment comprising:

a body contact electrode,
wherein the body contact electrode is placed on a back side of a chest of an animal.

[10] The garment for measuring biological information for animals according to [9], the garment comprising:

the body contact electrode,
wherein the body contact electrode is placed on a body part corresponding to at least one of a spinous process, a rib, and a scapula of the animal.

[11] The garment for measuring biological information according to [9] or [10],
wherein the electrode includes a stretchable conductor formed from at least a conductive filler and a flexible resin.
[12] The garment for measuring biological information according to [9] or [10],
wherein the electrode is formed from a conductive fiber material.
[13] The garment for measuring biological information according to any one of [9] to [12], further comprising:

a wiring electrically connected to the electrode; and
a connector for connecting a detachable electronic unit.

[14] The garment for measuring biological information according to any one of [9] to [13],
wherein the electrode is made of a material same as a material of the wiring in a seamless manner.
[15] The garment for measuring biological information according to any one of [9] to [14],
wherein biological information that is to be measured is an RR interval that is an interval between R waves in an electrocardiogram.
[16] A biological information measurement method for measuring biological information, the method comprising:
putting the garment for measuring biological information according to any one of [9] to [15] on an animal.
[17] The biological information measurement method according to [16],
wherein a conductive base material is applied to the electrode.
[18] A garment for measuring biological information for animals, the garment comprising, as constituent components, at least:

a garment body including a body contact electrode, a connector, and an electrical wiring that connects the electrode and the connector; and
an electronic unit detachable to the connector,
wherein the connector is located on a back side of a chest of an animal, the electrode and the electrical wiring are placeable on an arbitrary position in at least a back side area of the chest on an inner surface side of the garment, and the garment has a function of fastening the electrode and the electrical wiring on the arbitrary position in a detachable manner.

[19] The garment for measuring biological information for animals according to [18], the garment comprising:

the body contact electrode,
wherein the "arbitrary position in the back side area of the chest" includes a body part corresponding to at least one selected from a spinous process, a rib, and a scapula of the animal.

[20] The garment for measuring biological information according to [18] or [19],
wherein the electrode and/or the wiring includes a stretchable conductor formed from at least a conductive filler and a flexible resin.
[21] The garment for measuring biological information according to [18] or [19],
wherein the electrode and/or the wiring is formed from a conductive fiber material.
[22] The garment for measuring biological information according to any one of [18] to [21],
wherein the electrode is made of a material same as a material of the wiring in a seamless manner.
[23] The garment for measuring biological information according to any one of [18] to [22],
wherein biological information that is to be measured is an RR interval that is an interval between R waves in an electrocardiogram.
[24] The garment for measuring biological information according to any one of [18] to [23], further comprising:
an electronic unit supporting section for supporting the electronic unit on an inner side or on an outer side of the garment for measuring biological information.
[25] The garment for measuring biological information according to any one of [18] to [24],

wherein the function of fastening the electrode and/or the wiring on the arbitrary position on the inner side of the garment is achieved by a hook member of a hook-and-loop fastener.

[26] A biological information measurement method for measuring biological information, the method comprising: putting the garment for measuring biological information according to any one of [18] to [25] on an animal.

[27] The biological information measurement method according to [26], wherein a conductive base material is applied to the electrode.

Preferably, the present invention further includes the following configurations.

[28] A biological information measurement method including: measuring a cardiac electrical activity by attaching at least two of the electrode members of the present invention according to any one of [1] to [5] on a chest part of an H-shaped harness.

[29] The biological information measurement method according to [9], wherein the electrode is attached in a front-back direction in relation to a direction of a body of an animal to be measured.

[30] A biological information measurement method including: measuring a cardiac electrical activity by attaching at least two of the electrode members of the present invention according to any one of [1] to [5] on a figure-eight dog harness (harness).

[31] A biological information measurement method including: measuring a cardiac electrical activity by attaching at least two of the electrode members of the present invention according to any one of [1] to [5] to clothes covering at least a chest of a bipedal animal.

EFFECTS OF THE INVENTION

The present invention is used for measuring biological information remotely by a wearable measurement system. The present invention is mainly applied to measure biological information of animal phyla, is preferably applied to vertebrates, and is more preferably applied to vertebrates living on land. The present invention is more preferably applied to quadruped animals including mammals, reptiles, and amphibians. However, the present invention does not exclude application to bipedal animals (humans, apes, kangaroos, birds, etc.). The present invention is applicable to animals on which harnesses, belts, garments, and the like (hereinafter collectively referred to as clothes) can be put. The clothes may be newly designed so as to be easily applied to the present invention, but existing clothes can also be used. Harness is originally a horse tack, but nowadays, it is a collective term for equipment for attaching a leash to pets such as dogs. The present invention can be used in combination with an H-shaped harness, a figure-eight harness, or the like which is commonly sold for dogs. Clothes for dogs can be put on another animal, and used in combination with the present invention. Furthermore, the present invention can be used for humans in combination with underwear such as belts, bras, undershirts, and underpants.

The present invention provides an electrode member for measuring biological information that can be freely attached to and detached from clothes including existing harnesses. If the electrode member according to the present invention is used, the degree of freedom of the electrode installation position is significantly increased, and the position can be easily changed. Therefore, the electrode member can be used while determining the optimum position for measurement of biological information. In addition, the electrode member according to the present invention has high degree of freedom in size. Therefore, it is possible to cover from small animals such as small birds, hamsters, or small dogs to large animals such as large dogs, sheep, horses, cows, or elephants by preparing the electrode members of basic sizes.

The electrode member according to the present invention enables measurement without using an adhesive for a living body. Therefore, the electrode member can perform measurement without imposing an excessive load on a test subject, and also does not give pain even during removal. The present invention enables measurement of biological information of underwater animals such as dolphins, whales, crocodiles, turtles, hippopotamuses, sea lions, Steller's sea lions, fur seals, and penguins, if the shape of clothes is altered.

The present invention is not limited to such non-human animals, and can also be used for humans. For example, if the electrode member according to the present invention is attached to an edge part of an underwear such as a bra for women or an undershirt, a measurement system for measuring biological information can be implemented using clothes that are usually worn, whereby it is possible to monitor biological information in a natural condition.

The electrode member according to the present invention is easy to be attached and detached, and can be easily replaced when the electrode member is damaged or malfunctions.

In the present invention, particularly when a stretchable conductor is used for the electrode, fitting of the electrode section to the living body is improved, and appropriate moisture retaining effect is obtained. Therefore, biological information of a subject having long hair can also be satisfactorily measured.

The second and third aspects of the present invention are used for measuring biological information by a wearable measurement system. The second and third aspects are mainly applied to measure the biological information of animal phyla, are preferably applied to vertebrates, and are more preferably applied to vertebrates living on land. The second and third aspects are more preferably applied to quadruped animals including mammals, reptiles, and amphibians. The second and third aspects do not exclude application to bipedal animals (humans, apes, kangaroos, birds, etc.). The second and third aspects are applicable to animals on which harnesses, belts, garments, and the like (hereinafter collectively referred to as clothing) can be put. Garments may be newly designed so as to be easily applied to the second and third aspects, but existing garments can also be used. Harness is originally a horse tack, but nowadays, it is a collective term for equipment for attaching a leash to pets such as dogs. The second and third aspects can be used in combination with an H-shaped harness, a figure-eight harness, or the like which is commonly sold for dogs. Garments for dogs can be put on another animal, and used in combination with the second and third aspects.

The garments for measuring biological information according to the second and third aspects include electrodes on the back side of the chest, specifically, on a body part corresponding to the spinous process, the rib, and/or the scapula. Therefore, even when the animal is lying on its belly or on its side, the electrode is not sandwiched between the animal and the floor, the contact state between the electrode and the body does not change greatly, and displacement is unlikely to occur. Therefore, stable measurement of body information is enabled.

As a further effect provided by the second and third aspects, a body of a measurement section (preferably, a detachable electronic unit having a measurement function, a function of communicating with an outside, and the like) to be connected to the electrode can be mounted near the electrode, that is, on the back side of the chest of the animal, whereby, when the animal is lying on its belly or on its side, the electronic unit is not covered by the body of the animal. Therefore, according to the garment for measuring biological information and the biological information measurement method provided by the second and third aspects, data from the electronic unit can be accurately transmitted, and biological information can be measured with high accuracy in daily life.

Furthermore, the electronic unit is easily attached on the back side of the chest of the animal. Therefore, the body of the animal does not interfere with information reception, wireless control, and GPS signal reception, whereby a stable communication state can be maintained.

The garment for measuring biological information according to the present invention in the present invention includes an electrode on the back side of the chest, specifically, on an arbitrary position selected from body parts corresponding to the spinous process, the rib, and/or the scapula in a detachable manner. Therefore, even when the animal is lying on its belly or on its side, the electrode is not sandwiched between the animal and the floor, the contact state between the electrode and the body does not change greatly, and displacement is unlikely to occur. Therefore, stable measurement of body information is enabled.

As a further effect provided by the present invention, a body of a measurement section (preferably, a detachable electronic unit having a measurement function, a function of communicating with an outside, and the like) to be connected to the electrode can be mounted near the electrode, that is, on the back side of the chest of the animal, whereby, when the animal is lying on its belly or on its side, the electronic unit is not covered by the body of the animal. Therefore, according to the garment for measuring biological information and the biological information measurement method provided by the present invention, data from the electronic unit can be accurately transmitted, and biological information can be measured with high accuracy in daily life.

Furthermore, the electronic unit is easily attached on the back side of the chest of the animal. Therefore, the body of the animal does not interfere with information reception, wireless control, and GPS signal reception, whereby a stable communication state can be maintained.

Further, when the electrode is mounted as an electrode member in which the electrode, a wiring, and a connector are integrated, electrical elements can be attached to and detached from a body of a garment. This is convenient when the body of the garment is washed.

BRIEF DESCRIPTION OF THE DRAWINGS

Fig. 1 shows a top view and a sectional view illustrating an example of a structure of an electrode member according to the present invention.
Fig. 2 shows a top view and a sectional view schematically illustrating a state in which the electrode member according to the present invention is attached to a detachable electronic device (detachable device).
Fig. 3 is a schematic view illustrating a state in which the electrode member according to the present invention and the detachable electronic device (detachable device) are attached to belt-like clothes.
Fig. 4 is a development view of an inner surface (surface in contact with a living body) of an example of a garment for measuring biological information according to the present invention.
Fig. 5 is a development view of an outer surface (surface not in contact with a living body) of an example of the garment for measuring biological information according to the present invention.

Fig. 6 is a view of a dog wearing an example of the garment for measuring biological information according to the present invention, the development views of the garment being illustrated in Figs. 4 and 5.

Fig. 7 is a development view of an inner surface (surface in contact with a living body) of an example of a garment for measuring biological information according to the present invention.

Fig. 8 is a development view of an outer surface (surface not in contact with a living body) of an example of the garment for measuring biological information according to the present invention.

Fig. 9 is a view of a dog wearing an example of the garment for measuring biological information according to the present invention, the development views of the garment being illustrated in Figs. 7 and 8.

Fig. 10 shows a top view and a sectional view illustrating an example of the structure of an electrode member according to the present invention. The direction of a snap fastener (500) is different from that in the example in Fig. 1.

Fig. 11 is a sectional view illustrating an example of a state in which the electrode member illustrated in Fig. 10 is attached to a detachable electronic unit (detachable device).

Fig. 12 is a schematic view illustrating a state in which the electrode member and the detachable electronic unit (detachable device) are attached to a garment.

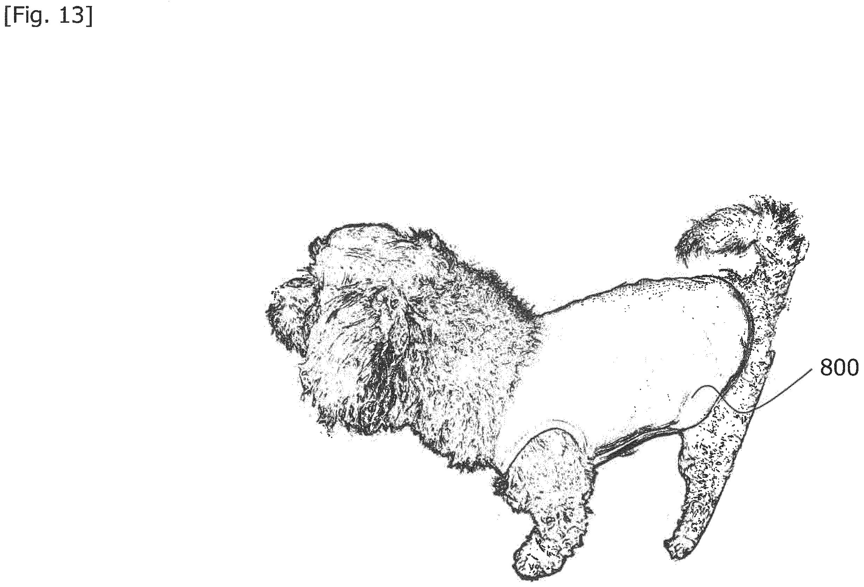

Fig. 13 is a schematic view illustrating a dog wearing the garment for measuring biological information according to the present invention.

Fig. 14 is a graph showing the value over time of RR interval of the heart rate of a dog obtained in Example of the present invention.

Fig. 15 is a graph showing the value over time of RR interval of the heart rate of a dog obtained in Comparative Example of the present invention.

MODE FOR CARRYING OUT THE INVENTION

The present invention will be described with reference to the drawings.

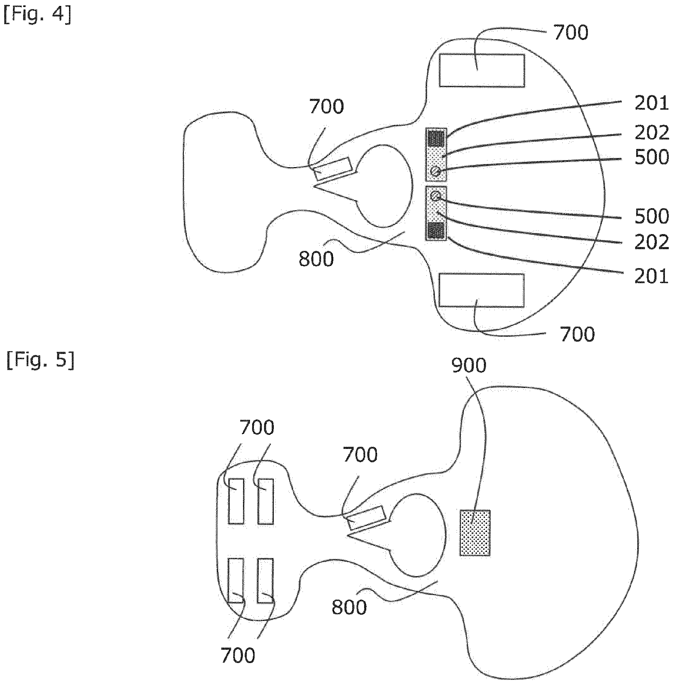

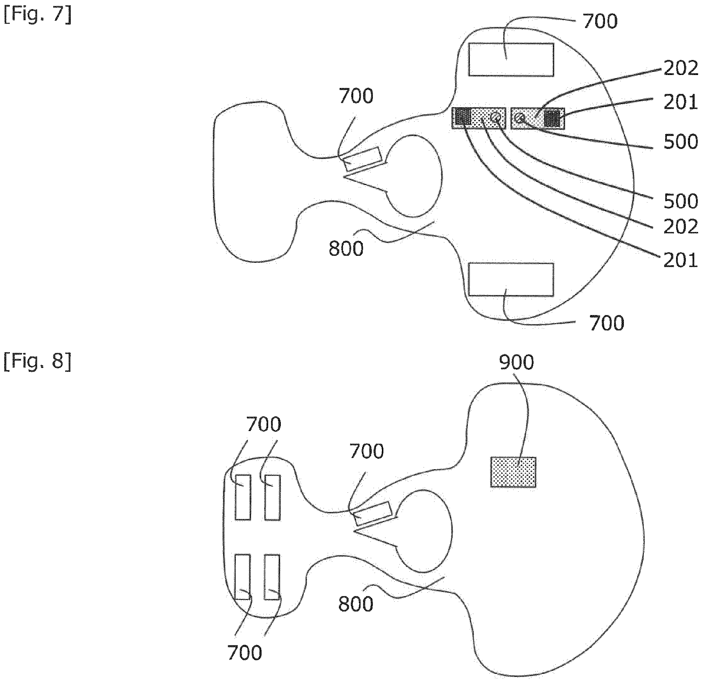

Figs. 4 and 7 are development views of a surface in contact with a body of an animal, that is, an inner surface, of an example of the garment for measuring biological information according to the present invention. When a dog is used as an example of the animal, an electrode member 101 having a sheet shape and including an electrode 201, a wiring 202, and a snap fastener 500 which are integrated is placed on the back side of the chest of the dog, specifically, on a body part corresponding to the spinous process, the rib, and/or the scapulae.

Figs. 5 and 8 are development views of a surface not in contact with the body of the animal, that is, an outer surface, of an example of the garment for measuring biological information according to the present invention. An electronic unit 900 is placed on the back side of the chest part of the garment 800 for measuring biological information, specifically on the body part corresponding to the spinous process, the rib, and/or the scapulae.

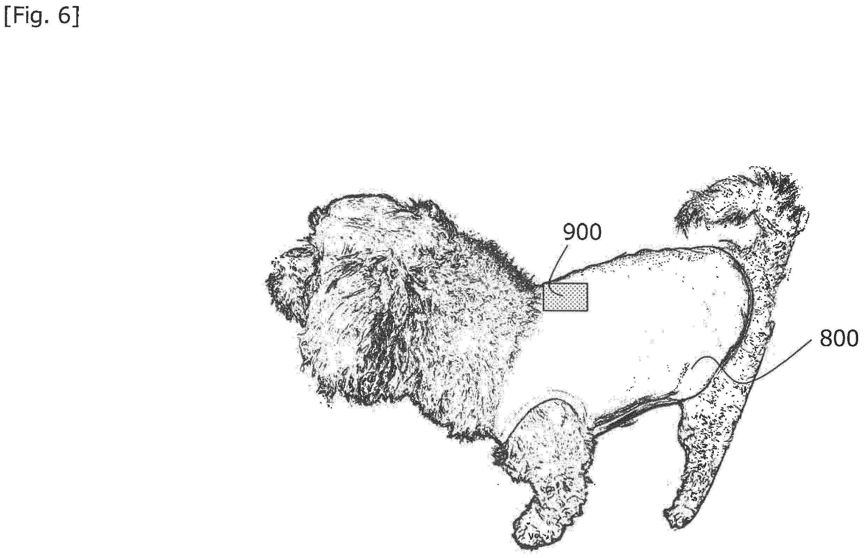

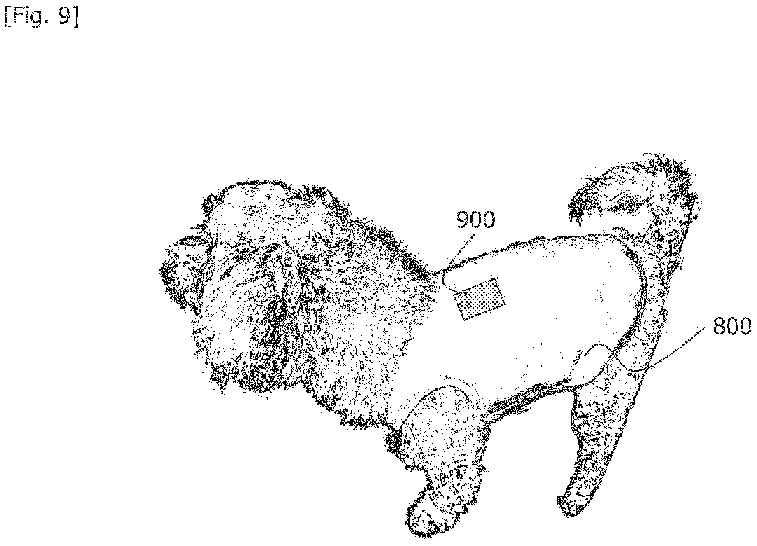

Figs. 6 and 9 are views illustrating a dog wearing the garment for measuring biological information according to the present invention. The electronic unit 900 is placed on the back side of the chest of the dog, specifically on the body part corresponding to the spinous process, the rib, and/or the scapulae.

The biological information in the present invention includes cardiac electrical activity, myoelectric activity, body temperature, temperature in the garment, respiratory rate, respiratory condition, amount of sweating, sweating stage, joint angle, displacement amount of each part of the body, acceleration rate of each part of the body, position information of each part of the body, etc., which can be detected by the electrode and/or another sensor. The electrode and/or the other sensor are appropriately selected according to biological information to be measured. Particularly, the present invention is preferably configured to include at least a plurality of electrodes that are in contact with a living body, and further include an electrode capable of measuring cardiac electrical activity as biological information. In general, the measurement result of cardiac electrical activity is recorded as an electrocardiogram and an electrocardiogram waveform in which the horizontal axis represents time and the vertical axis represents potential difference. The waveform that appears in the electrocardiogram for each heartbeat is mainly composed of five typical waves: P wave, Q wave, R wave, S wave, and T wave. There may be a U wave besides these waves. The wave from the start of the Q wave to the end of the S wave may be referred to as QRS wave. The present invention preferably includes an electrode capable of detecting at least the R wave from among these waves. The R wave shows the activation of both the left and right ventricles and is the wave with the largest potential difference. The time from the peak of the R wave to the peak of the next R wave is generally called RR interval (RRI), and the heart rate per minute can be calculated using the equation (heart rate) = 60/(RR interval (seconds)). That is, the heart rate can be found by detecting the R wave using an electrode capable of detecting the R wave. In the present invention, QRS waves are also included in R waves unless otherwise noted.

The following description is common to the first aspect, the second aspect, and the third aspect of the present invention, unless otherwise specified.

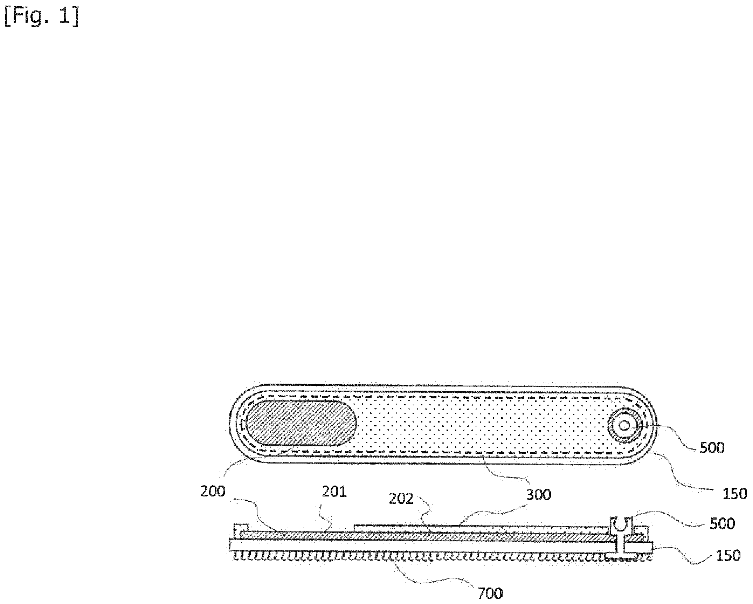

An example of the configuration of the present invention will be described with reference to the drawings. Fig. 1 shows a top view and a sectional view illustrating an example of a structure of an electrode member 100 according to the present invention. The electrode member 100 includes, on a first surface of a flexible base material 150, an electrode section 201, a wiring 202, and a snap fastener 500 used as a connector. Fig. 1 shows an example in which the electrode section and the wiring are continuously formed using the same flexible conductor 200. The wiring is insulated by a flexible insulator 300. In Fig. 1, a hook-and-loop fastener is attached to a second surface of the electrode member as an engagement member.

Examples of materials that can be used for the flexible base material 150 in the present invention include a PET film, a PEN film, a polyimide film, an elastomer film such as synthetic rubber, natural rubber, silicone rubber, and a urethane film, another polymer films, and a fabric made of a fiber material. Examples of the fabric include woven fabrics, knitted fabrics, and non-woven fabrics, and fabrics coated with resin and fabrics coated with and impregnated with resin can also be used for the flexible base material. A synthetic rubber sheet represented by chloroprene, etc. can also be used as the flexible base material. The fabric used in the present invention preferably has stretchability so that the fabric can repeatedly stretch 10% or more. The base material of the present invention preferably has a breaking elongation of 50% or more. The flexible base material in the present invention may be an original fabric, a ribbon, a tape, a braid, a net, or a piece of cloth cut from the original fabric.

When the fabric is a woven fabric, examples of weave include plain weave, twill weave, and satin weave. When the fabric is a knitted fabric, examples of knitting include flat knitting and variation thereof, moss stitch, amunzen, lace stitch, eyelet stitch, plating stitch, pile stitch, rib stitch, ripple stitch, hexagonal stitch, blister stitch, Milano rib stitch, double pique, single pique, twilling stitch, herringbone stitch, ponte stitch, entrelac knitting, tricot stitch, half tricot stitch, satin tricot stitch, double tricot stitch, queen's cord stitch, seersucker stripe stitch, Russell knitting, tulle mesh, and variation and combination thereof. The fabric may be a non-woven fabric made of elastomer fibers.

The fiber material in the present invention is not particularly limited. If the fiber material is a natural fiber, cotton, wool, linen, etc. can be used, and if the fiber material is a chemical fiber, nylon, acrylic, polyester, polyurethane, etc. can be used. These materials can be used alone or can be blended at an arbitrary ratio. The fiber material of the clothes for measuring biological information according to the present application preferably includes a cotton material in a weight ratio of 25% or more. More preferably, the fiber material includes a stretchable material in a weight ratio of 5% or more. Preferable examples of a blend material include a cotton-polyurethane blend material, and a cotton-polyester-polyurethane blend material. In particular, the fiber material of a section for supporting the electrode preferably includes cotton in a weight ratio of 35% or more.

Cloth made of the fiber material constituting the clothes according to the present invention may be either a woven fabric or a knitted fabric.

As a material used for the electrical wiring and/or the electrode in the present invention, a metal foil, a conductive fabric, a stretchable conductor sheet, and the like can be used. These materials can be used alone or in combination as appropriate. In the electrode portion, an electrode surface layer may be provided as necessary. The wiring portion is preferably covered with an insulating cover layer, preferably a stretchable insulating cover layer. Moreover, an underlayer for improving adhesiveness and achieving insulation may be provided on the boundary between the fabric and both the electrode and the wiring.

The metal foil used as the electrical wiring in the present invention is at least one or more metal foils having a thickness of 50 $\mu$m or less, preferably 25 $\mu$m or less, more preferably 15 $\mu$m or less, still more preferably 8 $\mu$m or less, and yet still more preferably 4 $\mu$m or less, and 0.08 $\mu$m or more and selected from copper foil, phosphor bronze foil, nickel plated copper foil, tin plated copper foil, nickel/gold plated copper foil, aluminum foil, silver foil, and gold foil.

These metal foils can be produced by conventional methods such as an electrolytic method, an electroless method, a rolling method, a vapor deposition method, and a sputtering method. Such metal foils can be processed into a predetermined pattern shape by an etching method, a lift-off method, an additive method, a punching method, a laser cutting method, or the like.

In the present invention, geometric redundancy means that, when a point A and a point B are defined in a space, these two points are connected using a path Y longer than the shortest distance X between the two points, by which the connection state is maintained with a margin even when the distance between the two points is increased.

Here, a redundancy factor is defined as follows.

$$\text{Redundancy factor} = \text{Y/X}$$

If a wide conductor pattern is used, the length here is the length of a line passing through the center of the conductor pattern.

In the present invention, the redundancy factor is preferably 1.41 or more, more preferably 1.8 or more, still more preferably 2.2 or more, and yet still more preferably 2.8 or more. In order to increase the redundancy factor, the metal foil may simply be arranged in zigzag, sinusoidally arranged, or arranged in a repeated horseshoe shape.

Such a metal foil preferably having a zigzag pattern is formed as follows, for example. A laminate of a metal foil and a stretchable sheet such as a rubber sheet, urethane sheet, or a silicone rubber sheet is formed, and then, an unnecessary portion of the metal foil is removed by a subtractive method to form a predetermined pattern. The subtractive method is synonymous with an etching method used in a method commonly used for manufacturing a printed wiring board. The stretchable sheet may also function as the underlayer or may function as a part of the underlayer.

When the metal foil is used as the electrode in the present invention, the surface of the metal foil is preferably plated with noble metal such as gold, silver, platinum, rhodium, or ruthenium, or plated with metal which is less susceptible to oxidation degradation due to passivation, such as chromium, molybdenum, tungsten, nickel, or a corrosion-resistant alloy. Thus, the surface of the metal foil can be protected. An electrode surface protective layer can also be provided by printing a carbon paste or the like on the surface of the electrode. Alternatively, the surface of the electrode can be covered with a stretchable conductive composition containing conductive particles and a flexible resin.

In the present invention, conductive yarns (including conductive threads and conductive fibers) can be used as the electrode or the electrical wiring. The wiring made of conductive yarns is preferably used in combination with an electrode using a conductive fabric. The conductive yarn in the present invention refers to a yarn having a resistance value of 100 $\Omega$ or less per 1 cm of fiber length. Here, the conductive yarn is a general term for conductive fibers, fiber bundles of conductive fibers, and twisted yarns, braided yarns, spun yarns, and blended yarns obtained from fibers containing conductive fibers. Examples of the conductive yarn in the present invention include a conductive yarn formed from: metal-coated chemical fiber; metal-coated natural fiber; chemical fiber coated with conductive oxide; natural fiber coated with conductive oxide; chemical fiber coated with carbon-based conductive material (graphite, carbon, carbon nanotube, graphene, etc.); natural fiber coated with carbon-based conductive material; chemical fiber coated with conductive polymer; and natural fiber coated with conductive polymer. The conductive yarn of this type includes a conductive extra-thin silt film obtained by forming a thin slit of 800 $\mu$m or less on a polymer film coated with one or more conductive materials selected from metal, carbon-based conductive material, conductive metal oxide, and conductive polymer.

In the present invention, it is possible to use a conductive yarn made of conductive fibers obtained by spinning a polymer having kneaded therein one or more conductive materials selected from metal, carbon-based conductive material, conductive metal oxide, and conductive polymer.

Further, in the present invention, it is possible to use metal microwires having a thickness of 250 $\mu$m or less, preferably 120 $\mu$m or less, still more preferably 80 $\mu$m or less, and yet still more preferably 50 $\mu$m or less as conductive fibers or conductive yarns. Furthermore, in the present invention, it is possible to use conductive yarns obtained by allowing a fiber bundle such as microfibers or nanofibers to carry conductive particles, conductive polymers, or the like, or by impregnating the fiber bundle with such particles, polymers, or the like.

In the present invention, it is particularly preferable to use at least one kind of conductive yarn selected from a metal-coated chemical fiber, a fiber bundle impregnated with a conductive polymer, and a metal microwire having a thickness of 50 $\mu$m or less.

It is preferable that the wiring formed from conductive yarns has redundancy. Redundancy can be given by a method such as embroidering the conductive yarn in a zigzag pattern, or inserting the conductive yarn into knit, creating a loop in the conductive yarn portion to ensure redundancy, and using the knit fabric as the wiring.

In the present invention, a conductive fabric can be used as the electrode or the electrical wiring. The conductive fabric in the present invention is a general term for a fibrous structure having electrical conductivity. Examples of the conductive fabric in the present invention include a woven fabric, a knitted fabric, and a non-woven fabric made of fibers containing conductive yarns (including conductive threads and conductive fibers). Moreover, in the present invention, the conductive fabric can be implemented by embroidering a non-conductive fabric with a conductive thread. Further, it is possible to use, as the conductive fabric, a fibrous structure obtained by impregnating a non-conductive fabric with a conductive polymer solution or a solution of a composition containing conductive particles and a binder resin, and drying the resultant.

It is preferable to use a fibrous structural object impregnated with a conductive polymer as the conductive fabric in the present invention. It is also preferable to use, as the conductive fabric in the present invention, a conductive fabric in which the fibrous structural object is impregnated with a conductive polymer by applying, to a fabric, a dispersion liquid prepared by dispersing the conductive polymer and a binder into a solvent medium. In the present invention, a mixture of poly(3,4-ethylenedioxythiophene) and polystyrene sulfonate can be preferably used as the conductive polymer. In the present invention, it is preferable that the conductive fabric used for the electrode be a woven or knitted fabric, and the basis weight of the woven or knitted fabric is less than 50 g/m$^2$ or more than 300 g/m$^2$. It is preferable that the fabric used for the electrode according to the present invention is composed of a synthetic fiber multifilament, and at least a part of the synthetic fiber multifilament is an ultrafine filament having a fineness of less than 30 dtex, or the synthetic fiber multifilament has a fineness exceeding 400 dtex, and has a single yarn fineness of 0.2 dtex or less.

In the present invention, a stretchable conductor layer (or a stretchable conductor layer sheet or simply a stretchable conductor layer) can be used as a material for the electrode and the electrical wiring. The stretchable conductor layer indicates a layer made of a material having stretchability and a specific resistance of $1 \times 10^{\circ}$ $\Omega$cm or less. The stretchable conductor layer in the present invention has stretchability. The stretchability in the present invention means that the conductor layer can repeatedly stretch by 10% or more while maintaining electrical conductivity. Further, the stretchable conductor layer alone in the present invention preferably has a breaking elongation of 40% or more, more preferably 50% or more, and still more preferably 80% or more. Further, the stretchable conductor layer in the present invention preferably has a tensile modulus of elasticity of 10 to 500 MPa. A material capable of forming such a stretchable conductor layer having stretchability is called a stretchable conductor composition.

The stretchable conductor composition can be obtained by using a conductive paste described below. Hereinafter, a conductive paste which is one of means for achieving the constituent elements of the present invention will be described. The conductive paste is composed of at least conductive particles, a flexible resin, and a solvent.

The conductive particle in the present invention is made of a substance having a specific resistance of $1 \times 10^{-1}$ $\Omega$cm or less and has a particle diameter of 100 $\mu$m or less. Examples of the substance having a specific resistance of $1 \times 10^{-1}$ $\Omega$cm or less include metal, alloy, carbon, doped semiconductor, and conductive polymer. Examples of the conductive particles preferably used in the present invention include: metals such as silver, gold, platinum, palladium, copper, nickel, aluminum, zinc, lead, or tin; alloy particles such as brass, bronze, white copper, and solder; hybrid particles such as silver-coated copper; metal-plated polymer particles; metal-plated glass particles; and metal-coated ceramic particles.

Preferred shapes of the metallic conductive particles include known flakes (scales), spheres, dendrites, aggregates (a shape wherein spherical primary particles are aggregated into a three-dimensional shape), and the like. Among these, metal powder in flakes, spheres or aggregates is particularly preferred.

In the present invention, it is preferable to use flaky silver particles or amorphous aggregated silver powder as the conductive particles. The particle diameter of the flaky powder is not particularly limited, but those having an average particle diameter (50% D) of 0.5 to 20 $\mu$m measured by a dynamic light scattering method are preferable. More preferably, the average particle diameter is 3 to 12 $\mu$m. If the average particle diameter exceeds 15 $\mu$m, it becomes difficult to form fine wiring, and clogging may occur when screen printing is applied. If the average particle diameter is less than 0.5 $\mu$m, the particles cannot contact each other at low filling, and electrical conductivity may be reduced. The particle diameter of the amorphous aggregated powder is not particularly limited, but those having an average particle diameter (50% D) of 1 to 20 $\mu$m measured by a light scattering method are preferable. More preferably, the average particle diameter is 3 to 12 $\mu$m. When the average particle diameter exceeds 20 $\mu$m, dispersibility is lowered and pasting becomes difficult. When the average particle diameter is less than 1 $\mu$m, the effect of the aggregated powder is lost, and excellent electrical conductivity may not be maintained at low filling.

The average particle diameter of the metal particles is preferably 0.5 to 10 $\mu$m. If the average particle diameter is too large, it may be difficult to form a desired pattern shape when the wiring is formed with a fine pattern. On the other hand, if the average particle diameter is too small, the metal particles tend to aggregate when the stretchable conductor layer is formed. Further, the raw material cost increases with a decrease in the particle diameter, which is not preferable.

The proportion of metal particles in the conductive particles is preferably 80% by volume or more, more preferably 85% by volume or more, and still more preferably 90% by volume or more. If the content of the metal particles is too small, it may be difficult to obtain sufficiently high electrical conductivity.

In the present invention, the volume% of each component is determined by measuring the mass of the solid content of each component contained in the paste, and calculating (mass of each solid content ÷ specific gravity of each solid content) to calculate the volume of the solid content of each component.

Preferable examples of the other conductive particles include carbon nanotubes. In particular, the conductive particle preferably has a mercapto group, an amino group, or a nitrile group on the surface, or is surface treated with rubber having a sulfide bond and/or a nitrile group. In general, a conductive material itself has a strong cohesive force, and a conductive material having a high aspect ratio has low dispersibility in resin. However, if the conductive material has a mercapto group, amino group, or nitrile group on the surface, or is surface treated with rubber having a sulfide bond and/or a nitrile group, the affinity for the metal particles is increased, so that an effective conductive network can be formed together with the metal particles. Thus, high electrical conductivity can be achieved.

The proportion of the conductive material in the conductive particles is preferably 20% by volume or less, more preferably 15% by volume or less, and still more preferably 10% by volume or less. If the content of the conductive material is too large, it may be difficult to uniformly disperse the conductive material in resin. Further, the conductive material described above is generally expensive. For these reasons, it is desired to reduce the use amount of the conductive material to the above-mentioned range.

Examples of metal nanoparticles include silver, bismuth, platinum, gold, nickel, tin, copper, and zinc, and the average particle diameter thereof is preferably 2 to 100 nm. In particular, from the viewpoint of electrical conductivity, copper, silver, platinum, and gold are preferable, and those containing silver and/or copper as a main component (50% or more by mass) are more preferable. Inclusion of metal nanoparticles can be expected to enhance electrical conductivity, and contributes to rheology control of the conductive paste used for forming the stretchable conductor layer, whereby printing property can be improved.

The proportion of the metal nanoparticles in the conductive particles is preferably 20% by volume or less, more preferably 15% by volume or less, and still more preferably 10% by volume or less. If the content of the conductive material is too large, the conductive material tends to easily aggregate in the resin. Further, the metal nanoparticles having a small diameter described above are usually expensive. For these reasons, it is desired to reduce the use amount of the metal nanoparticles to the above-mentioned range.

The amount of the conductive particles in the stretchable conductor layer (in other words, the amount of conductive particles in the total solid content of the conductive paste for forming the stretchable conductor layer) is preferably 15 to 45% by volume, and more preferably, 20 to 40% by volume. If the amount of the conductive particles is too small, the electrical conductivity may be insufficient. On the other hand, if the amount is too large, the stretchability of the stretchable conductor layer tends to decrease, and when the obtained stretchable electrode and wiring sheet are stretched, cracks or the like may be generated. As a result, satisfactory electrical conductivity may not be maintained.

Examples of the flexible resin in the present invention include a thermoplastic resin, a thermosetting resin, and rubber which have an elastic modulus of 1 to 1000 MPa. It is preferable that the flexible resin includes at least rubber containing a sulfur atom and/or rubber containing a nitrile group. A sulfur atom and a nitrile group have a high affinity with metals, and rubber is highly stretchable and can prevent generation of cracks and the like even when stretched. Therefore, even when the electrode and the wiring sheet are stretched, the conductive particles are held in a uniformly dispersed state, whereby excellent electrical conductivity can be provided. Rubber containing a nitrile group is more preferable from the viewpoint of a variation in electric resistance when being stretched. Note that the resins described as examples for forming the stretchable conductor layer may be used alone or in combination.

The rubber containing a sulfur atom is not particularly limited as long as it is rubber or elastomer containing sulfur. The sulfur atom can be contained in a form such as a sulfide bond or disulfide bond in a main chain of a polymer or as a mercapto group in a side chain or terminal of a polymer. Specific examples of the rubber containing a sulfur atom include polysulfide rubber, polyether rubber, polyacrylate rubber, and silicone rubber which contain a mercapto group, a sulfide bond, or a disulfide bond. In particular, polysulfide rubber, polyether rubber, polyacrylate rubber, and silicone rubber which contain a mercapto group are preferable. It is also possible to use a resin obtained by blending a sulfur-containing compound such as pentaerythritol tetrakis(S-mercaptobutyrate), trimethylolpropane tris(S-mercaptobutyrate), or mercapto group-containing silicone oil into rubber having no sulfur atom. Preferable examples of a commercially available product that can be used as rubber containing a sulfur atom include "Thiokol (registered trademark) LP" which is a liquid polysulfide rubber and which is manufactured by Toray Fine Chemical Co., Ltd. The content of the sulfur atom in the rubber containing a sulfur atom is preferably 10 to 30% by mass.

The rubber containing a nitrile group is not particularly limited as long as it is rubber or elastomer containing a nitrile group. Preferably, acrylonitrile butadiene copolymer rubber which is a copolymer of butadiene and acrylonitrile is used. Preferable examples of a commercially available product that can be used as rubber containing a nitrile group include "Nipol (registered trademark) 1042" manufactured by Zeon Corporation. The amount of the nitrile group in the rubber containing a nitrile group (particularly, the amount of acrylonitrile in the acrylonitrile butadiene copolymer rubber) is preferably 18 to 50% by mass, and more preferably 28 to 41% by mass. When the amount of bonding acrylonitrile in the acrylonitrile butadiene copolymer rubber increases, the affinity to metal increases, but the rubber elasticity contributing to stretchability decreases conversely.

The flexible resin that is a constituent component of the stretchable conductor layer is preferably composed only of rubber containing a sulfur atom and rubber containing a nitrile group. However, another resin other than rubber containing a sulfur atom and rubber containing a nitrile group may be included as long as electrical conductivity, stretchability, application property during the formation of the stretchable conductor layer, and the like are not impaired. When the other resins are included, the total amount of the rubber containing a sulfur atom and the rubber containing a nitrile group in all the resins is set to be preferably 95% by mass or more, more preferably 98% by mass or more, and still more preferably, 99% by mass or more.

The content of the above-mentioned resin in the stretchable conductor layer (in other words, the amount of the resin solid content in the total solid content of the conductive paste for forming the stretchable conductor layer) is preferably 55 to 85% by volume, and more preferably 60 to 80% by volume. When the content of the resin is too small, there is a tendency that the electrical conductivity may increase, but the stretchability may deteriorate. If the content of the resin is too large, there is a tendency that the stretchability may improve, but the electrical conductivity may decrease.

Additionally, examples of usable rubber include urethane rubber, acrylic rubber, silicone rubber, butadiene rubber, nitrile group-containing rubber such as nitrile rubber and hydrogenated nitrile rubber, isoprene rubber, sulfurized rubber, styrene butadiene rubber, butyl rubber, chlorosulfonated polyethylene rubber, ethylene propylene rubber, and vinylidene fluoride copolymer.

In the present invention, an underlayer can be provided as necessary. The underlayer is also referred to as a first insulating layer, and is located between the base material and the stretchable conductor layer. The underlayer in the present invention provides insulation on the base material side of the wiring. Here, the insulation includes mechanical, chemical, and biological insulation in addition to electrical insulation, and indicates a function of insulating the conductor layer from moisture, chemical substances, and biological substances that permeate the base material.

The underlayer in the present invention is preferably made of a flexible polymer material. As the flexible polymer material, a material called rubber or elastomer can be used. As the rubber and elastomer in the present invention, the resin materials described as examples of the flexible resin used for the stretchable conductor composition can be preferably used.

The underlayer in the present invention preferably has stretchability so that the underlayer can repeatedly stretch 10% or more. The underlayer in the present invention preferably has a breaking elongation of 50% or more. Further, the underlayer in the present invention preferably has a tensile modulus of elasticity of 10 to 500 MPa.

The underlayer in the present invention is preferably applied to the base material in a liquid form such as a coating liquid, an immersion liquid, a printing ink, or a printing paste, or a slurry state. It is also possible to form in advance an underlayer in the form of sheet in a separate process and bond the sheet-shaped underlayer to the fabric by a method such as hot pressing.

(First insulating layer)

The underlayer (first insulating layer) serves as a bonding surface when the electrode and the wiring sheet are laminated on the base material, and prevents moisture from reaching the stretchable conductor layer from the side opposite to the side of the base material on which the first insulating layer is laminated, when the electrode member is worn. Although the stretchable conductor layer described later in the present invention has good stretchability, if the base material is made of a material having excellent stretchability exceeding the stretchability of the stretchable conductor layer, the stretchable conductor layer may be stretched following the elongation of the base material, resulting in generation of cracks. The first insulating layer also serves as an elongation stopper to suppress, when being laminated on a garment, the elongation of the garment and prevent the stretchable conductor layer from being excessively elongated.

The resin used for forming the first insulating layer is not particularly limited, as long as it has insulating property. Preferable examples of the resin include polyurethane resin, silicone resin, vinyl chloride resin, epoxy resin, and polyester elastomer. Among others, polyurethane resin is preferable from the viewpoint of adhesiveness to the stretchable conductor layer. Note that the resins described as examples for forming the first insulating layer may be used alone or in combination. A reactive hot melt material can also be used as the first insulating layer. For example, a bonding sheet using a B-stage flexible epoxy resin used for a flexible printed wiring board can also be used.

The first insulating layer in the present invention can be formed in such a way that the insulating resin described above is dissolved or dispersed in an appropriate solvent (preferably, water), the resultant is applied or printed on a release paper or a release film to form a coating film, and then, the solvent contained in the coating film is volatilized and dried. The later-described commercially available sheet or film having appropriate physical properties can also be used.

The thickness of the first insulating layer is preferably 5 to 200 $\mu$m, and more preferably 12 to 120 $\mu$m. If the first insulating layer is too thin, the insulating effect and the elongation stopping effect may become insufficient. On the other hand, if the first insulating layer is too thick, the stretchability is impaired, and the thickness of the electrode and the wiring tends to increase, which may deteriorate wear comfort, when the first insulating layer is laminated on the garment.

(Second insulating layer)

In the present invention, when the surface of the stretchable conductor layer is covered for insulation, a second insulating layer (also referred to as an insulating cover layer) can be formed on the stretchable conductor layer. This configuration can prevent the stretchable conductor layer from being in contact with water such as rain or sweat, when a biological information measurement interface produced using the stretchable wiring sheet is worn.

The resins described as resins for forming the first insulating layer are used as resins for forming the second insulating layer, and preferable resins are also the same as those for forming the first insulating layer. The resins described as examples for forming the second insulating layer may also be used alone or in combination. The resin for forming the first insulating layer and the resin for forming the second insulating layer may be the same or different, but it is preferable to use the same resin from the viewpoint of performance for covering the stretchable conductor layer and reduction in damage of the stretchable conductor layer due to imbalanced stress when the wiring sheet is stretched. As described above, the second insulating layer can be formed in the same manner as the first insulating layer. The later-described commercially available sheet or film having appropriate physical properties can also be used.

The thickness of the second insulating layer is preferably 5 to 200 $\mu$m, and more preferably 150 to 20 $\mu$m. If the second insulating layer is too thin, the second insulating layer is apt to deteriorate due to the repeated expansion and contraction of the base material, resulting in that the insulating effect becomes insufficient. On the other hand, if the second insulating layer is too thick, the stretchability of the wiring sheet may be impaired, or the thickness of the entire wiring tends to increase, resulting in that wear comfort may be deteriorated.

When the stretchable conductor layer according to the present invention is used for the electrode to be in contact with a living body, a moisture retention layer may be provided in order to reduce contact impedance with the living body. Further, a gel electrode layer may be provided to further reduce and stabilize the contact impedance.

The engagement member in the present invention is not particularly limited in means and material thereof as long as it has a function for temporarily fastening the electrode member to clothes. Examples of the engagement member include an adhesive tape and a hook-and-loop fastener. In addition, the electrode member can be attached to clothes using a button, a snap fastener, a fastener, etc. in combination in place of a means having a surface engagement function. Furthermore, the electrode member may be simply tied to clothes with a string or may be attached using a rubber band.

The electrical wiring in the present invention is preferably covered with a flexible insulating layer. The flexible insulating layer covers the electrical wiring using an insulating cover coat layer. Similar to the underlayer, the cover coat layer can be made of a stretchable resin material or the like.

The connector section in the present invention is not particularly limited as long as it is a connector that can be attached to the flexible base material in the present invention and can be electrically connected to the wiring. In the present invention, a metal snap fastener can be used as the connector. In the present invention, the wiring is connected to the detachable electronic device 900 via the connector. In one example of the present invention, a base part of the snap fastener is in contact with the wiring, and a protruding part of the snap fastener can be connected to the detachable electronic device, as a preferred example. If the detachable electronic device does not have sufficient water resistance, it is removed from clothes when the clothes are washed, and only the clothes are washed. However, if the electronic device has sufficient waterproof function, it is not necessary to design the electronic device to be detachable. In that case, a connection tool that does not require attachment and detachment of the connector section may be used.

The detachable electronic device according to the present invention (detachable device, also referred to as a detachable electronic unit) is a compact electronic unit having a function of receiving and measuring at least an electrical signal obtained from an electrode in contact with a living body via a wiring and a connector, and more preferably, having a calculation function, a display function, a storage function, and a communication function. In the present invention, it is preferable to use a detachable electronic device having a function of performing AD conversion of a biological signal and communicating with an external device.

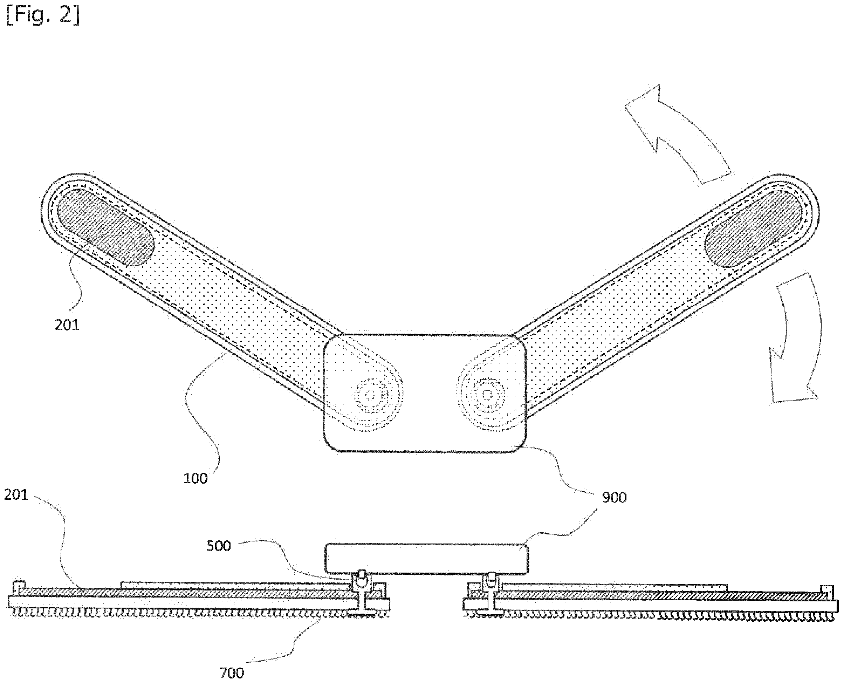

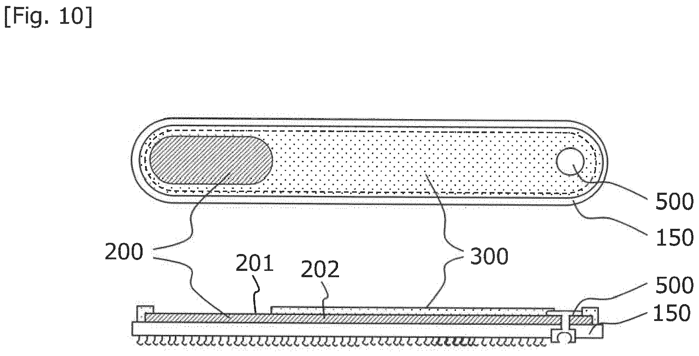

The electrode member according to the present invention is connected to the detachable electronic device as shown in Figs. 2 and 10, for example. As indicated by block arrows in the figures, the angle with respect to the detachable electronic device can be freely changed.

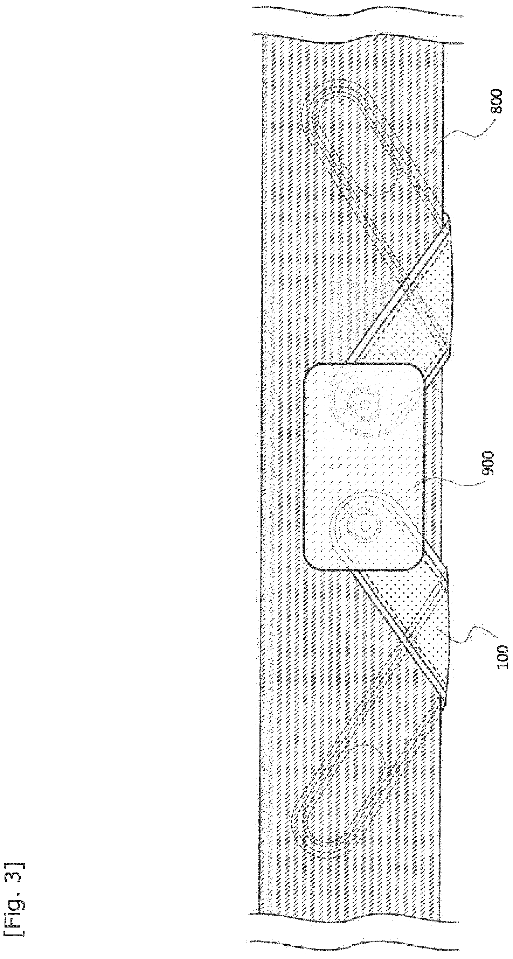

As shown in Fig. 3 as an example, the electrode member according to the present invention can be attached by folding the electrode member so as to wrap the edge of clothes. The electrode position and the attachment position of the detachable electronic device can be freely adjusted by the way to fold the electrode member, whereby the biological information measurement system can be attached to the subject using existing clothes by appropriately adjusting the attachment position of the detachable electronic device, while determining an electrode position appropriate for the measurement of biological information. If several types of electrode members having different dimensions such as length and width are prepared, any types of subjects such as small-sized animals and animals having relatively a large body can be appropriately measured. It is obvious that, even when the subject is a human, the biological information can be measured by similarly attaching the system to existing clothes (for example, underwear).

The size of the electrode member according to the present invention is not particularly limited, but can be appropriately adjusted within the range of 20 mm to 1500 mm inclusive in the longitudinal size. The longitudinal size of the electrode member according to the present invention is preferably within a range from 40 mm or more to 300 mm or less, and more preferably within a range from 60 mm or more to 200 mm or less.

In the present invention, when, for example, two electrode members with a longitudinal size of 80 mm $\pm$ 10 mm, two electrode members with 120 m $\pm$ 15 mm, and two electrode members with 180 mm $\pm$ 20 mm, that is, six electrode members in total, are prepared as one set, it is possible to measure biological information of popular pet animals mainly including dogs and cats.

The second and third aspects of the present invention provide a garment for measuring biological information for animals, the garment including a body contact electrode, wherein the body contact electrode is placed on the back side of the chest of an animal. The body contact electrode is positioned on a body part corresponding to at least one selected from the spinous process, the rib, and the scapula of the animal, and the electrode member according to the first aspect of the present invention can be used as a means for providing a degree of freedom in an electrode position.

The third aspect of the present invention is an aspect obtained by further improving the second aspect. In the example described in the second aspect, the electronic unit is exposed to the outside of the garment. However, in the present invention, the electronic unit can be placed inside the garment.

An example of the configuration of the present invention will be described with reference to the drawings. The electrode member 100 has, on a first surface of the flexible base material 150, a wiring 202 for electrically connecting a single electrode section 201 made of a flexible conductive material and a snap fastener 500 used as a connector section, the snap fastener 500 being mounted on a second surface of the flexible substrate. The wiring is insulated by a flexible insulator 300. In the drawings, a hook-and-loop fastener 700 is attached to the electrode member as an engagement member.

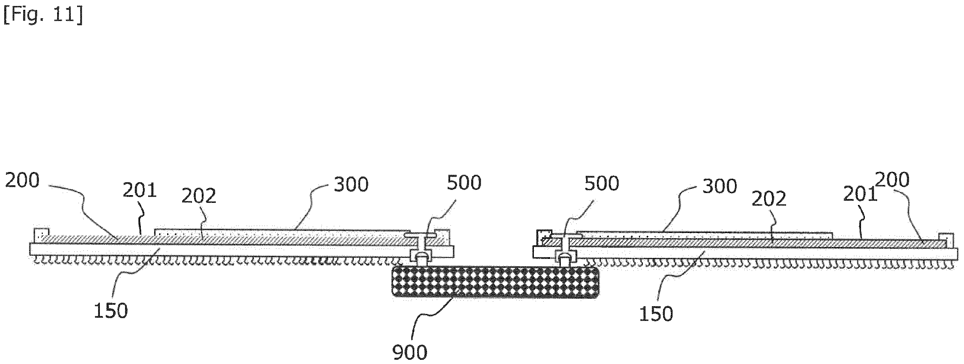

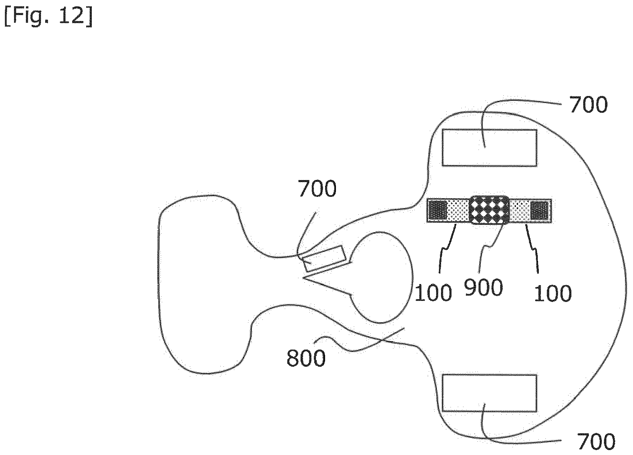

Fig. 10 is a sectional view illustrating an example of a state in which the electrode member 100 according to the present invention is attached to the detachable electronic unit 900. This electrode member is affixed to the inner side of the garment body while being adjusted to be located on the back side of the chest of an animal, preferably on a body part corresponding to the spinous process, the rib, and/or the scapula, as shown in Fig. 11, and the garment is put on the animal as shown in Fig. 12. Thus, even when the animal is lying on its belly or on its side, the electrode section and the electronic unit are not sandwiched between the animal and the floor, so that they are unlikely to displace. Accordingly, the biological information can be accurately measured in daily life. Furthermore, when the animal is lying on its belly or on its side, the electronic unit is not covered by the body of the animal, whereby transmission means of the electronic unit such as radio waves and infrared rays can be accurately transmitted.

Fig. 10 shows that the electronic unit and the electrode member are directly connected. However, the electronic unit and the electrode member may be indirectly connected via a connector provided on the garment body. The garment body preferably has a pocket for preventing the electronic unit from falling, and the pocket may be provided either on the outside or on the inside (body contact side) of the garment body. A connector terminal may be provided in the pocket, this connector terminal may be electrically connected to a connector terminal outside the pocket, and the connecter terminal outside the pocket may be connected to the electrode member.

The connector used in the present invention is preferably a connector having a degree of freedom of rotation such as a snap fastener. When the electrode member is attached to the connector with a degree of freedom of rotation, the electrode position can be changed in the circumferential direction, and the arrangement position of the electrode can be freely changed by flexing the electrode member or using electrode members having different lengths.

It is preferable that a hook part of the hook-and-loop fastener is provided on the surface of the electrode member opposite to the surface on which the electrode is provided. With this configuration, the electrode member can be fastened to the inside of the garment body in a detachable manner. Note that the means for fastening the electrode member is not limited to the hook-and-loop fastener, and a self-adhesive tape or an adhesive material may be used. A configuration in which the connector side of the electrode member is fastened to the garment body is also within the scope of the present invention.

The biological information in the first, second, and third aspects are common to the present invention. In addition, as in the present invention, it is also preferable to combine the first, second, and third aspects with a detachable electronic unit having a measurement function, communication function, and the like besides the electrode.

The garment for measuring biological information according to the present invention includes at least a garment body, a single electrode made of a conductive material, a single-pole connector section, and a wiring that electrically connects the electrode and the connector section, the electrode and the wiring being preferably made of the same material in order to reduce a level difference and ensure connection reliability. Although not particularly limited, the garment for measuring biological information according to the present invention includes: an electrode provided on the inner surface of the garment body in contact with a living body; a connector section and an electronic unit provided on an outer surface not in contact with the living body; and a wiring that connects the electrode and the wiring. The distance between the connector and the electrode is preferably as short as possible. In the present invention, the electrode is provided on the back side of the chest, so that the connector and the electronic unit can be easily mounted on the back side. As a result, even when the animal is lying on its belly or on its side, the electrode and the electronic unit are not sandwiched between the animal and the floor, which prevents displacement, prevents the animal from having an uncomfortable feeling, and prevents a great variation in a contact pressure between the electrode and the body. Further, when the animal is lying on its belly or on its side, the electronic unit is not covered by the body of the animal, whereby the transmission/reception function of the electronic unit is unlikely to be impaired.

The garment for measuring biological information according to the present invention includes at least a garment body, a single electrode made of a conductive material, a single-pole connector section, and a wiring that electrically connects the electrode and the connector section, the electrode and the wiring being preferably made of the same material in order to reduce a level difference and ensure connection reliability. Although not particularly limited, the garment for measuring biological information according to the present invention includes: an electrode provided on the inner surface of the garment body in contact with a living body; a connector section and an electronic unit provided on an outer surface not in contact with the living body; and a wiring that connects the electrode and the wiring. The distance between the connector and the electrode is preferably as short as possible. In the present invention, the electrode is provided on the back side of the chest, so that the connector and the electronic unit can be easily mounted on the back side. As a result, even when the animal is lying on its belly or on its side, the electrode and the electronic unit are not sandwiched between the animal and the floor, which prevents displacement, prevents the animal from having an uncomfortable feeling, and prevents a great variation in a contact pressure between the electrode and the body.

Further, when the animal is lying on its belly or on its side, the electronic unit is not covered by the body of the animal, whereby the transmission/reception function of the electronic unit is unlikely to be impaired.

In the present invention, the electrode preferably includes a conductive layer capable of detecting electrical information of a living body, and further includes an insulating layer. The electrode has an area necessary for detecting electrical information of a living body such as an electrocardiogram, and the area of each electrode is preferably 1 cm$^2$ or more. More preferably, it is 5 cm$^2$ or more, and still more preferably 10 cm$^2$ or more. The upper limit is not particularly limited, but is preferably 100 cm$^2$ or less. The electrode can be formed into any shape such as a rectangle, a triangle, a polygon with five or more sides, a circle, and an ellipse.

It is preferable that the electrode and/or the wiring in the present invention have stretchability so that they can follow the movement of the subject being measured.

As the conductor layer of the electrode and/or the electrical wiring in the garment for measuring biological information according to the present invention, a metal foil, conductive fibers, conductive yarns, and a stretchable conductor layer can be used, for example. Examples of usable metal foil include copper foil, aluminum foil, stainless steel foil, gold foil, and silver foil. Examples of usable conductive fibers or conductive yarns include a metal thin wire, metal-coated natural fibers or synthetic fibers, fibers made of a polymer material containing conductive particles such as metal microparticles or carbon nanofibers, and fibers coated with or impregnated with a conductive polymer. In addition, felts, fabrics, knitted fabrics, non-woven fabrics, and the like composed of these conductive fibers or conductive yarns can also be used as the conductor layer of the electrical wiring.

The stretchability can be achieved by a knit structure made of conductive fibers or conductive yarns, zig zag embroidery, or the like. A woven fabric also has stretchability in the bias direction of warp/weft. If a woven fabric, a knitted fabric, a non-woven fabric, or the like is formed of stretchable yarns, stretchability can be naturally obtained. The metal foil can be simulated by being processed into a corrugated shape.

In the present invention, stretchability can be obtained by using a sheet-shaped electrode and/or wiring formed from a stretchable conductive composition containing conductive particles and a resin having stretchability.

The sheet-shaped electrode formed from the stretchable conductive composition is preferable, because components having high electrical conductivity such as metal particles can be used for the sheet-shaped electrode, by which electric resistance lower than that when a conductive polymer is used can be obtained, and thus, a weak electric signal can be detected. The electric resistance value on the electrode surface is preferably 1000 Ω□ or less, more preferably 300 Ω□ or less, still more preferably 100 Ω□ or less, and most preferably 30 Ω□ or less in terms of sheet resistance. In the sheet-shaped electrode formed from the conductive composition, the electric resistance value on the electrode surface can be suppressed to 300 Ω□ or less.

In addition, since the electrode formed from the conductive composition has a low electric resistance value, the wiring and the electrode can be made of the same material, which is a preferable mode of the present invention. When the wiring and the electrode are made of the same material, the width of the wiring may be 1 mm or more, and more preferably 5 mm or more and 10 mm or less.

In the present invention, it is preferable to measure biological information using a garment provided with at least two or more electrodes. Although not particularly limited, the number of electrodes is preferably 2 or more and 15 or less. The form of the garment is not particularly limited, and the garment according to the present invention is applied to a garment that can be put on animals, such as a harness, a strap, or a garment (hereinafter collectively referred to as garment). The garment may be newly designed so as to be easily applied to the present invention, but existing garments can also be used. Harness is originally a horse tack, but nowadays, it is a collective term for equipment for attaching a leash to pets such as dogs. The present invention can be used in combination with an H-shaped harness, a figure-eight harness, or the like which is commonly sold for dogs. Garments for dogs can be put on another animal, and used in combination with the present invention. The garment is provided with two or more electrodes, and biological information can be measured by putting the garment.

The electrode used in the present invention preferably further has an insulating layer. For example, a sheet-shaped electrode including a first insulating layer and a stretchable conductor layer can be used. A sheet-shaped wiring including a first insulating layer, a stretchable conductor layer, and a second insulating layer can be used for the wiring used in the present invention.

Hereinafter, an example of forming a sheet-shaped electrode material which is one of preferable modes of the present invention and which includes an electrode and a wiring integrally formed from the same material will be described.

The stretchable conductor layer in the present invention can be formed in such a way that a composition (conductive paste) prepared by dissolving or dispersing each component described above in an appropriate organic solvent is directly applied or printed on the first insulating layer in a desired pattern to form a coating film, and then, the organic solvent contained in the coating film is volatilized and dried. Alternatively, a conductive paste may be applied or printed on release sheet or the like to form a coating film, the organic solvent contained in the coating film may be volatilized and dried to form a sheet-shaped stretchable conductor layer in advance, and the stretchable conductor layer may be laminated on the first insulating layer in a desired pattern.

The conductive paste can be prepared by uniformly dispersing conductive particles in resin using, as appropriate, a conventionally known method for dispersing powder in a liquid. For example, after metal particles, a dispersion liquid of a conductive material, and a resin solution are mixed, the resultant may be uniformly dispersed by an ultrasonic method, a mixer method, a three-roll mill method, a ball mill method, or the like. These methods can be used in combination.

The method for applying or printing the conductive paste is not particularly limited. Examples of the method include coating, screen printing, offset litho printing, an ink jet method, flexographic printing, gravure printing, gravure offset printing, stamping, dispensing, and squeegee printing.

In order to volatilize and dry the organic solvent after the coating film is formed using the conductive paste, the coating film may be heated, for example, in the air, in a vacuum atmosphere, in an inert gas atmosphere, or in a reductive gas atmosphere. The heating temperature may be selected in the range of, for example, 20 to 200°C in consideration of required electrical conductivity, heat resistance of the base material and the insulating layer, and the like.

The thickness of the dried stretchable conductor layer is preferably 8 to 150 $\mu$m, and more preferably 12 to 90 $\mu$m. If the stretchable conductor layer is too thin, it is likely to deteriorate due to repeated expansion and contraction of the electrode and the wiring sheet, so that electrical continuity may be interfered or blocked. On the other hand, when the stretchable conductor layer is excessively thick, the stretchability of the base material is impaired and the total thickness of the electrode and the wiring is increased, which may reduce wear comfort.

In a preferred embodiment of the stretchable electrode and the wiring sheet used in the present invention, a load per unit width during stretch at a stretch rate of 10% is 100 N/cm or less, more preferably 80 N/cm or less, and still more preferably 50 N/cm. In a conventional conductive fabric and wiring, a load per unit width during stretch at a stretch rate of 10% is 100 N or more, so that they hardly follow the elongation of the base material. Therefore, wear comfort when worn may be reduced. On the other hand, the stretchable electrode and the wiring sheet according to the present invention have a feature capable of suppressing the load per unit width during stretch at a stretch rate of 10% to 100 N/cm or less due to the use of rubber containing a sulfur atom and/or rubber containing a nitrile group as a resin for forming the stretchable conductor layer. The details of the stretch-load test in the present invention are described in Examples.

In a preferred embodiment of the stretchable electrode and the wiring sheet according to the present invention, an electric resistance change rate when the stretchable electrode and the wiring sheet are stretched by 20% is 5 times or less, more preferably 4 times or less, and still more preferably 3 times or less. In general, a conventional conductive fabric and wiring may be broken until being stretched at a stretch rate of 20%, or even when they can be stretched at a stretch rate of 20%, electrical conductivity decreases significantly to such an extent that the electric resistance change rate exceeds 10 times. In contrast, the stretchable electrode and the wiring sheet according to the present invention have a feature capable of suppressing the electric resistance change rate to 5 times or less when being stretched at 20% due to the use of rubber containing a sulfur atom and/or rubber containing a nitrile group as a resin for forming the stretchable conductor layer. The details of the stretching test in the present invention are described in Examples.

In a preferred embodiment, the thickness of the stretchable electrode and the wiring sheet according to the present invention is 400 $\mu$m or less, more preferably 300 $\mu$m or less, and still more preferably 200 $\mu$m or less. A conventional conductive fabric and wiring have a thickness of 400 $\mu$m or more, and tend to give a wearer a feeling of a foreign body when in contact with the skin. On the other hand, the stretchable electrode and the wiring sheet according to the present invention have a feature capable of suppressing the thickness to 400 $\mu$m or less while keeping high electrical conductivity due to the stretchable conductor layer formed from conductive particles mainly containing metal particles and rubber containing a sulfur atom and/or rubber containing a nitrile group as a resin.

The electrode and the wiring sheet according to the present invention can be laminated on a base material such as a garment. It is preferable to laminate the electrode and the wiring sheet with the first insulating layer facing the base material. The lamination method is not particularly limited as long as it is a conventionally known lamination method such as laminating with an adhesive or laminating by hot pressing. From the viewpoints of fitting to the body when worn for measurement of biological information and followability during exercise and movement, a lamination method that does not impair the stretchability of the electrode and the wiring sheet is preferable.

(Garment for measuring biological information)

The garment for measuring biological information according to the present invention is provided with an electrode and/or an electronic unit which are placed on the back side of the chest of an animal, preferably on a body part corresponding to the spinous process, the rib, and/or the scapula, when put on the animal, the electrode and the electronic unit being connected to each other via a wiring and a connector.

The biological information measurement method and the garment for measuring biological information according to the present invention preferably include an electronic unit that measures the biological information of a subject being measured by the electrode and a mechanism that analyzes the measured information.

As the mechanism for analyzing the measured information, a conventionally known analysis device (such as a heart rate meter, an electrocardiograph, or an electromyograph) according to purposes may be employed, and the mechanism includes a unit for transmitting information to an external analysis device.

The biological information measurement method and the garment for measuring biological information according to the present invention are also applicable to a technique for detecting a physiological state and a psychological state of animals on the basis of the collected biological information or in combination with a biological information measurement method and a garment for measuring biological information different from those of the present invention. Examples of such techniques include management of the psychoneurotic state of animals by measuring a degree of relaxation, and management of the health state by measuring the heart rate.

The biological information measurement method and the garment for measuring biological information according to the present invention and the second and third aspects enable measurement of biological information in such a way that the garment for measuring biological information is put on an animal, a conductive base material is applied on the electrode, and the electrode is brought into close contact with the body of the animal. Nonlimiting examples of the conductive base material include a conductive gel, a conductive paste, and a conductive jelly. Examples of commercially available product of the conductive paste include singa gel (Parker Laboratories, INC) and Ten20 (Weaver and Company). The electrode in the present invention may be provided with a moisture retention layer in order to reduce contact impedance with a living body.

EXAMPLES

Examples will be described below.

[Example 1]

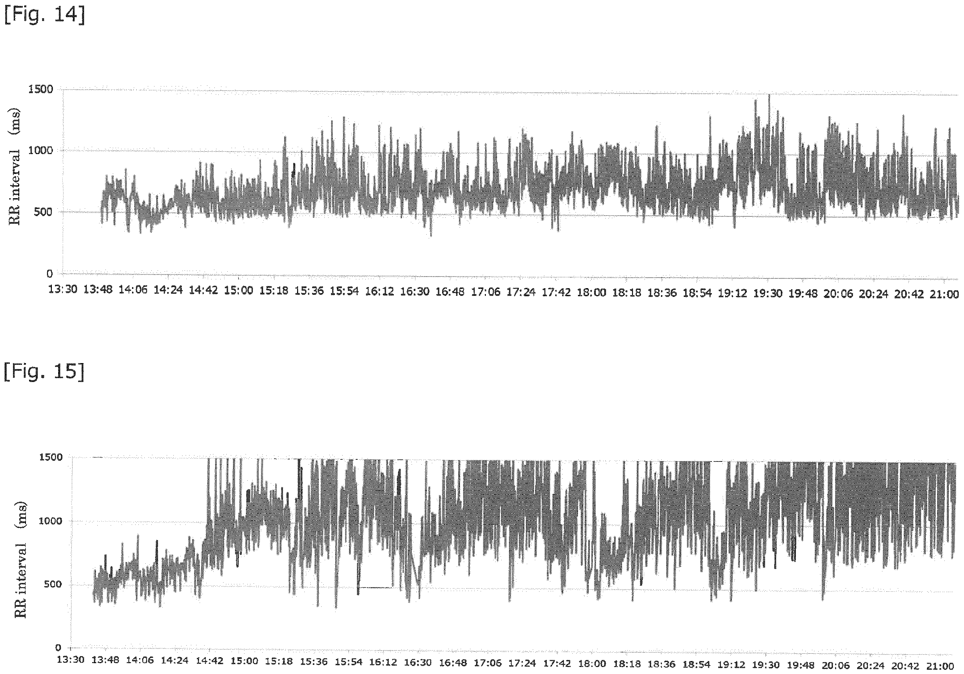

The electrode member shown in Fig. 10 was connected to a detachable unit having electrical specifications compatible with WHS-1 (manufactured by Union Tool) as shown in Fig. 11, and attached to the garment for measuring biological information shown in Fig. 12, singa gel (Parker Laboratories, INC) was applied to the electrode section, the garment was put on a Chihuahua (female, 3 years old), and the RR interval was measured in daily life. The result is shown in Fig. 14. In the figure, the horizontal axis indicates time (time of day), and the vertical axis indicates RR interval (ms). The result indicates that a variation in the RR interval could be stably measured continuously from 14:00 to 21:00 on the day when the measurement was started, as shown in Fig. 14.

[Comparative Example 1]

As a comparative example, a garment for measuring biological information provided with an electrode section located on the part corresponding to an axilla was produced, singa gel (Parker Laboratories, INC) was applied to the electrode section, the garment was put on a Chihuahua (female, 3 years old), and the RR interval was measured in daily life. The result is shown in Fig. 15. As shown in Fig. 15, the RR interval could be measured at the beginning of the measurement, but a time slot where the measurement was impossible increased, and much noise was recorded after 16:00. The observation of the behavior of the Chihuahua indicates that hours when she was lying on her belly or on her side increased after 16:00. It is considered that, for this reason, the electrode or the electronic unit displaced from the living body because the Chihuahua was lying on her belly or on her side, by which time slots when the measurement was impossible increased, and much noise was recorded.

[Example 2]

The garment for measuring biological information shown in Figs. 4 and 5 was produced, singa gel (Parker Laboratories, INC) was applied to the electrode section, the garment was put on a Chihuahua (female, 3 years old) as shown in Fig. 6, and the RR interval was measured in daily life using a detachable unit having electrical specifications compatible with WHS-1 (manufactured by Union Tool) as an electronic unit. The result indicates that a variation in the RR interval could be stably measured continuously from 14:00 to 21:00 on the day when the measurement was started.

[Example 3]

The garment for measuring biological information shown in Figs. 7 and 8 was produced, singa gel (Parker Laboratories, INC) was applied to the electrode section, the garment was put on a Chihuahua (female, 3 years old) as shown in Fig. 9, and the RR interval was measured in daily life in the same manner as in Example 2. The result indicates that a variation in the RR interval could be stably measured continuously from 14:00 to 21:00 on the day when the measurement was started.

INDUSTRIAL APPLICABILITY

As described above, the electrode member according to the present invention has an excellent characteristic of easily assembling a system optimum for measurement of biological information by being mounted on an arbitrary position of existing clothes, while determining an appropriate electrode position and attachment section of the electronic device, regardless of the size and shape of a subject. The present invention can be applied to pet animals such as mice, hamsters, dogs, cats, reptiles, amphibians, or birds, domestic animals such as horses, cows, or sheep, wild animals, and humans.

Furthermore, the garment for measuring biological information and the biological information measurement method to which the present invention is applied prevent the electrode and the electronic unit from being sandwiched between an animal and the floor, even when the animal is lying on its belly or on its side, and thus, displacement is unlikely to occur. Therefore, biological information can be accurately measured in daily life. In addition, when the animal is lying on its belly or on its side, the electronic unit is not covered by the body of the animal, whereby data can be accurately transmitted from the electronic unit. Thus, the biological information can be obtained with high accuracy.

The present specification has described the present invention, mainly taking dogs as an example for convenience. However, the present invention is not limited to dogs. The present invention is preferably applicable to vertebrates including aquatic mammals, aquatic reptiles, and fish. The present invention is also more preferably applicable to vertebrates living on land, and still more preferably to quadruped animals including mammals, reptiles, and amphibians. In addition, the present invention does not exclude application to bipedal animals (humans, apes, kangaroos, birds, etc.). The present invention is widely usable for health control of a subject (test subject), study and research involving wild animals, and a communication tool with pets.

DESCRIPTION OF REFERENCE SIGNS

| | |
|---|---|
| 100: | electrode member |
| 150: | flexible base material |
| 200: | flexible conductor |
| 201: | electrode section |
| 202: | wiring |
| 300: | flexible insulator |
| 500: | snap fastener |
| 700: | hook-and-loop fastener |
| 800: | clothes |
| 900: | electronic device (detachable device) |

**Claims**

1.    A garment for measuring biological information for animals, the garment comprising:

a body contact electrode,
wherein the body contact electrode is placed on a back side of a chest of an animal.

2.    The garment for measuring biological information for animals according to claim 1, the garment comprising:

the body contact electrode,
wherein the body contact electrode is placed on a body part corresponding to at least one of a spinous process, a rib, and a scapula of the animal.

3.    The garment for measuring biological information according to claim 1 or 2,
wherein the electrode includes a stretchable conductor formed from at least a conductive filler and a flexible resin.

4. The garment for measuring biological information according to claim 1 or 2,
   wherein the electrode is formed from a conductive fiber material.

5. The garment for measuring biological information according to any one of claims 1 to 4, further comprising:

   a wiring electrically connected to the electrode; and
   a connector for connecting a detachable electronic unit.

6. The garment for measuring biological information according to any one of claims 1 to 5,
   wherein the electrode is made of a material same as a material of the wiring in a seamless manner.

7. The garment for measuring biological information according to any one of claims 1 to 6,
   wherein biological information that is to be measured is an RR interval that is an interval between R waves in an electrocardiogram.

8. A biological information measurement method for measuring biological information, the method comprising:
   putting the garment for measuring biological information according to any one of claims 1 to 7 on an animal.

9. The biological information measurement method according to claim 8,
   wherein a conductive base material is applied to the electrode.

[Fig. 1]

[Fig. 2]

[Fig. 3]

[Fig. 4]

[Fig. 5]

EP 4 052 649 A1

[Fig. 6]

800

900

[Fig. 7]

[Fig. 8]

[Fig. 9]

[Fig. 10]

[Fig. 11]

[Fig. 12]

[Fig. 13]

EP 4 052 649 A1

[Fig. 14]

[Fig. 15]

31

Europäisches
Patentamt

European
Patent Office

Office européen
des brevets

**PARTIAL EUROPEAN SEARCH REPORT**

under Rule 62a and/or 63 of the European Patent Convention.
This report shall be considered, for the purposes of
subsequent proceedings, as the European search report

**Application Number**

**EP 22 15 0339**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/326387 A1 (BELALCAZAR ANDRES [US] ET AL) 31 December 2009 (2009-12-31) * paragraphs [0001], [0002], [0004], [0031]; claim 1; figure 1 * ----- | 1,2,8,9 | INV. A61B5/27 A61B5/00 A01K29/00 |
| X | WO 2017/085403 A1 (@HEALTH [FR]) 26 May 2017 (2017-05-26) * page 1 lines 1-3; page 5 lines 10-18; page 8 lines 19-24; page 8 line 30 – page 9 line 2; page 11 line 27 – page 12 line 2; figures 3A-3C, 5A * ----- | 1,3,7 | ADD. A61D9/00 |
| X | US 2010/198043 A1 (HOLZER CHRISTIAN [DE] ET AL) 5 August 2010 (2010-08-05) * paragraphs [0002], [0041], [0045], [0049], [0050]; claims 1, 4, 5, 6, 7, 11; figures 1a-1c * ----- | 1,4-6 | |
| X | JP 2002 035141 A (HICHI YASUTAKE; OMURA TORYO KK) 5 February 2002 (2002-02-05) * paragraphs [0001], [0031]; claims 1, 5, 6; figure 3 * ----- | 1,2 | TECHNICAL FIELDS SEARCHED (IPC) A61B A61D A01K |

## INCOMPLETE SEARCH

The Search Division considers that the present application, or one or more of its claims, does/do not comply with the EPC so that only a partial search (R.62a, 63) has been carried out.

Claims searched completely :

Claims searched incompletely :

Claims not searched :

Reason for the limitation of the search:

**see sheet C**

| Place of search | Date of completion of the search | Examiner |
|---|---|---|
| The Hague | 28 July 2022 | Fauché, Yann |

EPO FORM 1503 03.82 (P04E07)

Europäisches
Patentamt
European
Patent Office
Office européen
des brevets

INCOMPLETE SEARCH
SHEET C

Application Number

EP 22 15 0339

Claim(s) completely searchable:
    –


Claim(s) searched incompletely:
    1-9


Reason for the limitation of the search:

1  An invitation pursuant to Rule 63(1) EPC was sent dated 13-04-2022 outlining a lack of clarity (Art. 84 EPC) of independent claim 1.
2  Answering to this invitation with letter filed on 30-05-2022, the applicant provided a set of claim-like statements further defining, on the basis e.g. of paragraph 15, the relationship between the electrode and the garment body by indicating that the electrode is affixed to an inner side of the garment body so that the garment as a whole is suitable for being used with the electrode being placed on a back side of a chest of an animal.
3  This further indication indeed makes clear that protection is thought for a garment+electrode which would be suitable for the claimed use.
4  The search has therefore been based on the statement indicating the subject-matter to be searched submitted by the applicant on 30-05-2022 and limited to this subject-matter.
----------------------------------------------------------

## ANNEX TO THE EUROPEAN SEARCH REPORT
## ON EUROPEAN PATENT APPLICATION NO.

EP 22 15 0339

This annex lists the patent family members relating to the patent documents cited in the above-mentioned European search report.
The members are as contained in the European Patent Office EDP file on
The European Patent Office is in no way liable for these particulars which are merely given for the purpose of information.

28-07-2022

| Patent document cited in search report | | Publication date | Patent family member(s) | | Publication date |
|---|---|---|---|---|---|
| US 2009326387 | A1 | 31-12-2009 | NONE | | |
| WO 2017085403 | A1 | 26-05-2017 | AU | 2016355074 A1 | 05-07-2018 |
| | | | BR | 112018010013 A2 | 21-11-2018 |
| | | | CA | 3005277 A1 | 26-05-2017 |
| | | | CN | 108471956 A | 31-08-2018 |
| | | | EP | 3376943 A1 | 26-09-2018 |
| | | | FR | 3043902 A1 | 26-05-2017 |
| | | | JP | 2018534118 A | 22-11-2018 |
| | | | RU | 2018122091 A | 30-12-2019 |
| | | | US | 2018333058 A1 | 22-11-2018 |
| | | | WO | 2017085403 A1 | 26-05-2017 |
| US 2010198043 | A1 | 05-08-2010 | DE | 102008051536 A1 | 15-04-2010 |
| | | | EP | 2177156 A1 | 21-04-2010 |
| | | | US | 2010198043 A1 | 05-08-2010 |
| JP 2002035141 | A | 05-02-2002 | NONE | | |

EPO FORM P0459

For more details about this annex : see Official Journal of the European Patent Office, No. 12/82

**REFERENCES CITED IN THE DESCRIPTION**

**Patent documents cited in the description**

- JP 2006141467 A
- WO 2017026416 A